# EUROPEAN PATENT SPECIFICATION

(11) **EP 3 302 604 B1**
(45) Date of publication and mention of the grant of the patent: **11.01.2023**
(21) Application number: 16729699.5
(22) Date of filing: 27.05.2016
(51) Int. Cl.: A61M 1/14, A61M 1/16

(54) **SENSOR CALIBRATION FOR DIALYSIS SYSTEMS**
SENSORKALIBRIERUNG FÜR DIALYSESYSTEME
ÉTALONNAGE DE CAPTEURS POUR SYSTÈMES DE DIALYSE

(30) Priority: 02.06.2015 US 201514728611
(43) Date of publication of application: 11.04.2018
(73) Proprietor: Fresenius Medical Care Holdings, Inc., Waltham, MA 02451-1457 (US)
(72) Inventor: WANG, Aiyuan, San Ramon, California 94582 (US); WANG, Fei, Concord, California 94521 (US); CRNKOVICH, Martin Joseph, Walnut Creek, California 94598 (US)
(74) Representative: Fish & Richardson P.C.
(86) International application number: PCT/US2016/034742
(87) International publication number: WO 2016/196325

(56) References cited:
- EP-A1- 0 846 470
- EP-A2- 1 938 847
- US-A- 4 662 208
- US-A- 5 872 454
- US-A1- 2011 244 578

## Description

### TECHNICAL FIELD

This disclosure relates to sensor calibration for dialysis systems.

### BACKGROUND

Renal dysfunction or failure and, in particular, end-stage renal disease, causes the body to lose the ability to remove water and minerals and excrete harmful metabolites, maintain acid-base balance and control electrolyte and mineral concentrations within physiological ranges. Toxic uremic waste metabolites, including urea, creatinine, and uric acid, accumulate in the body's tissues which can result in a person's death if the filtration function of the kidney is not replaced.

Dialysis is commonly used to replace kidney function by removing these waste toxins and excess water. In one type of dialysis treatment--hemodialysis--toxins are filtered from a patient's blood externally in a hemodialysis machine. Blood passes from the patient through a dialyzer separated by a semi-permeable membrane from a large volume of externally-supplied dialysis solution. The waste and toxins dialyze out of the blood through the semi-permeable membrane into the dialysis solution, which is then discarded.

The dialysis solutions or dialysates used during hemodialysis typically contain sodium chloride and other electrolytes, such as calcium chloride, or potassium chloride, a buffer substance, such as bicarbonate, or acetate and acid to establish a physiological pH, plus optionally, glucose or another osmotic agent.
EP1938847 (A2) describes a method for checking the operating configuration of the arterial and venous lines of a blood treatment apparatus. The blood treatment apparatus includes a blood treatment unit having a semi permeable membrane delimiting a first chamber through which blood removed from said blood access passes and a second chamber through which dialysis liquid passes, an arterial line connected to an inlet of the first chamber, and a venous line connected to an outlet of the first chamber, said arterial and venous lines being able to be configured according to at least a normal configuration, in which said arterial line carries blood from said upstream position of said blood access, and said venous line carries blood towards said downstream position of said blood access, and to at least a reversed configuration, in which said arterial line carries blood from said downstream position of said blood access, and said venous line carries blood towards said upstream portion of said blood access.
US 5,872,454 describes a a two-point calibration procedure that allegedly corrects for non-linear behavior of conductivity cells.

US 2011/244578 discloses a device for calibrating a conductivity sensor, wherein a calibration curve is computed based on conductivity measurements taken by the sensor and a reference device.

### SUMMARY

The present invention provides a dialysis system according to claim 1 and a method for calibrating a conductivity sensor according to claim 8.

Systems and methods of calibrating conductivity sensors of a dialysis system can include several features as described herein. The systems and methods can be used to calibrate the conductivity sensors using fluids of unknown concentrations. The conductivity sensors can be calibrated to two or more target measurements of the conductivity. Pump flow rates can be adjusted during calibration to maintain conductivities within predefined ranges. The systems and methods can adjust flow rates an amount proportional to a difference between the target measurement and a measurement of the conductivity sensor.

In one aspect, a dialysis system including at least one pump, a conductivity meter, at least one conductivity detector, and a controller. The at least one pump disposed along a fluid circuit conducts or is configured to conduct a fluid at a pump rate through the fluid circuit, wherein the pump rate is adjustable. The conductivity meter includes a probe configured to be placed in fluid communication with the fluid circuit to measure conductivity values of the fluid. The at least one conductivity detector is disposed along the fluid circuit to measure sensor values of the fluid. The controller is configured to receive the conductivity values from the conductivity meter and the sensor values from the at least one conductivity detector, compute a plurality of representative conductivity values from the received conductivity values and a plurality of representative sensor values from the received sensor values, determine a first conductivity point associated with a first representative conductivity value and a first representative sensor value, determine a second conductivity point associated with a second representative conductivity value and a second representative sensor value; and compute a calibration curve based on the first and second calibration points.

In some cases, the dialysis system can include a dialyzer connected to the fluid circuit.

In some examples, the controller can be configured to adjust a pump rate of the at least one pump until the first representative conductivity value is within a calibration interval before the controller determines the first conductivity point. The controller can be configured to introduce a delay after the pump rate adjustment and before the first conductivity point determination. The delay can be greater than 30 seconds. In some examples, the calibration interval is defined by an upper threshold and a lower threshold.

Alternatively or additionally, the controller can configured to adjust a pump rate of the at least one pump until the second representative conductivity value is within a calibration interval before the controller determines the second conductivity point. The controller can be configured to introduce a delay after the pump rate adjustment and before the second conductivity point determination. The controller can be configured to introduce a delay after the pump rate adjustment and before the second conductivity point determination. The delay can be greater than 30 seconds. The calibration interval can be defined by an upper threshold and a lower threshold.

In some implementations, the dialysis system can include a temperature sensor disposed along the fluid circuit. The controller can be configured to adjust the conductivity value of the at least one conductivity detector based on a temperature measured by the temperature sensor. The controller is configured to compute the calibration curve via at least one of linear interpolation and linear extrapolation.

In some examples, the controller can be configured to determine a third calibration point. The controller can be configured to compute the calibration curve based on the first, second, and third calibration points. The controller can be configured to compute the calibration curve via a nonlinear polynomial fit of the first, second, and third calibration points.

Another aspect includes a method for calibrating at least one conductivity detector disposed along a dialysis fluid circuit. The method includes passing a fluid through the dialysis fluid circuit by running at least one pump disposed along the dialysis fluid circuit, receiving conductivity values from a conductivity meter disposed along the dialysis fluid circuit and sensor values from the at least one conductivity detector disposed along the dialysis fluid circuit, and computing a plurality of representative conductivity values from the received conductivity values and a plurality of representative sensor values from the received sensor values. The method further includes determining a first conductivity point associated with a first representative conductivity value and a first representative sensor value, determining a second conductivity point associated with a second representative conductivity value and a second representative sensor value, and computing a calibration curve based on the first and second calibration points.

In some examples, the dialysis fluid circuit further includes a dialyzer.

The method can include, before determining the first conductivity point, adjusting a pump rate of the at least one pump until the first representative conductivity value is within a calibration interval. The method can include introducing a delay after adjusting the pump rate and before determining the first conductivity point. The delay can be greater than 30 seconds. The calibration interval can be defined by an upper threshold and a lower threshold.

In some cases, the method can include, before determining the second conductivity point, adjusting a pump rate of the at least one pump until the second representative conductivity value is within a calibration interval. The method can include introducing a delay after adjusting the pump rate and before determining the second conductivity point. The delay can be greater than 30 seconds. The calibration interval can be defined by an upper threshold and a lower threshold.

In some examples, at least one of the first and second representative conductivity values can be a moving average of the received conductivity values. In some the at least one of the first and second representative sensor values is a moving average of the received sensor values.

The method can further include receiving temperature values from a temperature sensor disposed along the fluid circuit. The method can include adjusting the conductivity value of the at least one conductivity detector based on a temperature measured by the temperature sensor. The conductivity detector and temperature sensor can be a single sensor.

In some cases, computing the calibration curve can include at least one of a linear interpolation and a linear extrapolation.

In some implementations, the method can include determining a third calibration point. Computing the calibration curve can be based on the first, second, and third calibration points. The calibration curve further can include a nonlinear polynomial fit of the first, second, and third calibration points.

In some implementations of the systems and methods described herein, the fluid can be a solution. The solution can be of unknown concentration. The solution can include salt. The solution can include an acid. The at least one pump can be configured to conduct a salt solution, an acid solution, and/or a buffer solution.

In some implementations of the systems and methods described herein, the at least one conductivity detector can include a conductivity detector disposed along a path of the fluid circuit configured to be upstream of a dialyzer. The at least one conductivity detector disposed along a path of the fluid circuit configured to be downstream of the dialyzer. The at least one conductivity detector can include a conductivity detector disposed along a path of the fluid circuit in between a salt injection port in the fluid circuit and an acid solution injection port in the fluid circuit.

Implementations can include one or more of the following advantages. In some implementations, the calibration of the conductivity detectors is an automated process that reduces opportunity for user error. During non-automated calibration, a user typically selects a solution of known conductivity and manually calibrates the conductivity detectors to that known conductivity. The conductivity corresponds to a concentration of salts in the solution. Error can occur in selecting the solution of known conductivity, manually inputting the pre-selected conductivity into the calibration software, and/or manually inputting the conductivity detector output into the calibration software. In certain methods described herein, the user starts the calibration process, and the system automatically modulates the concentration of the dialysate solution until the solution reaches a target conductivity as measured by a calibrated conductivity meter. The system receives both the measurement from the conductivity meter and the conductivity detector and calibrates the detector to that point. The only user intervention that occurs is to start the program. The automated process allows a single user to calibrate multiple machines at once and improves the consistency of the calibration process between machines.

In some implementations, the calibration of the conductivity detectors is a two-point calibration where the two target conductivities are selected based upon the use cases of the hemodialysis machine. During a one-point calibration, a user assumes a detector read-out of zero to correspond to a conductivity of zero. The user then only has to calibrate the detector to a single conductivity that lies in the vicinity of the range expected during use. Conversely, a two-point calibration allows a user to select points that bracket the expected conductivity during use. Selecting points that bracket the conductivity typically seen during use reduces conductivity detector output error, such as error caused by detector nonlinearity, during treatment.

In some implementations, the automated calibration process can include adjustments to system parameters that are adaptive. For example, the default settings upon starting the calibration process may result in a measured conductivity that is far from the target conductivity point. The magnitude of this difference can determine the magnitude of the system adjustment such that the system reaches the target conductivity point faster with reduced overshoot. The adaptive feedback can increase the speed of calibration.

The automated calibration processes described herein can also reduce error associated with the salt and acid solution concentrations. The process can use salt solution (e.g., a buffer solution) and acid solution sources of unknown concentrations of salt and acid, respectively. The salt and acid solutions do not need to be precisely and accurately prepared before calibrating, and the risk of a user selecting an incorrect salt or acid solution is eliminated.

Other aspects, features, and advantages will be apparent from the description and drawings, and from the claims.

### DESCRIPTION OF DRAWINGS

FIG. 1 depicts a hemodialysis system that is connected to a patient.
FIG. 2 is a schematic diagram of a dialysate circuit and associated components of the hemodialysis system of FIG. 1.
FIG. 3 depicts a hemodialysis system of FIG. 1 during calibration of conductivity detectors shown in FIG. 2.
FIG. 4 is a flow chart of a two-point calibration process for the conductivity detectors shown in FIG. 2.
FIG. 5 is a graph of an example output from the two-point calibration process set forth in the flow chart of FIG. 4.
FIG. 6 is a graph of an example transient response of the conductivity detectors of FIG. 2.

### DETAILED DESCRIPTION

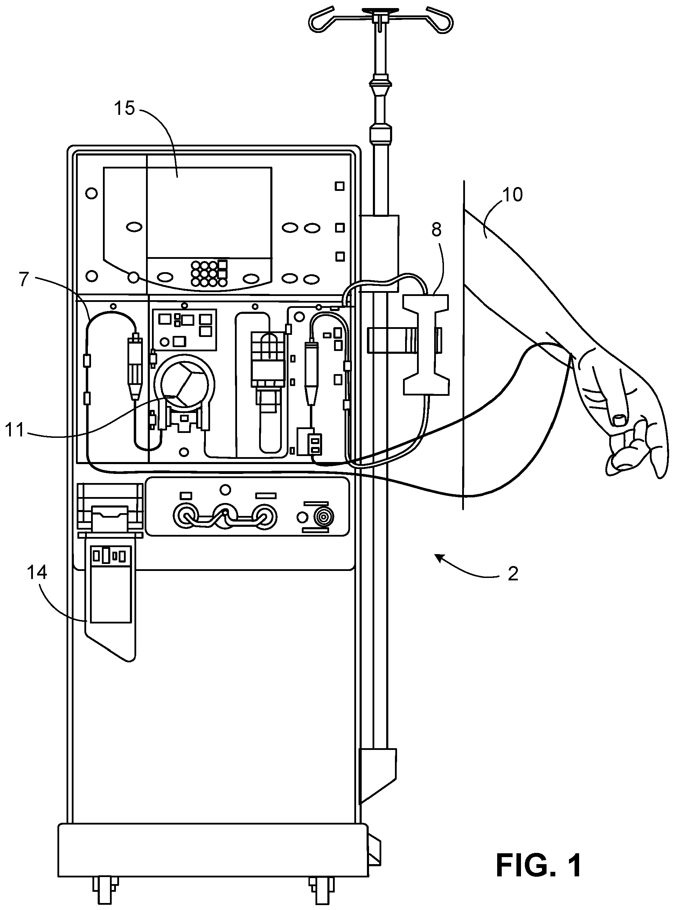

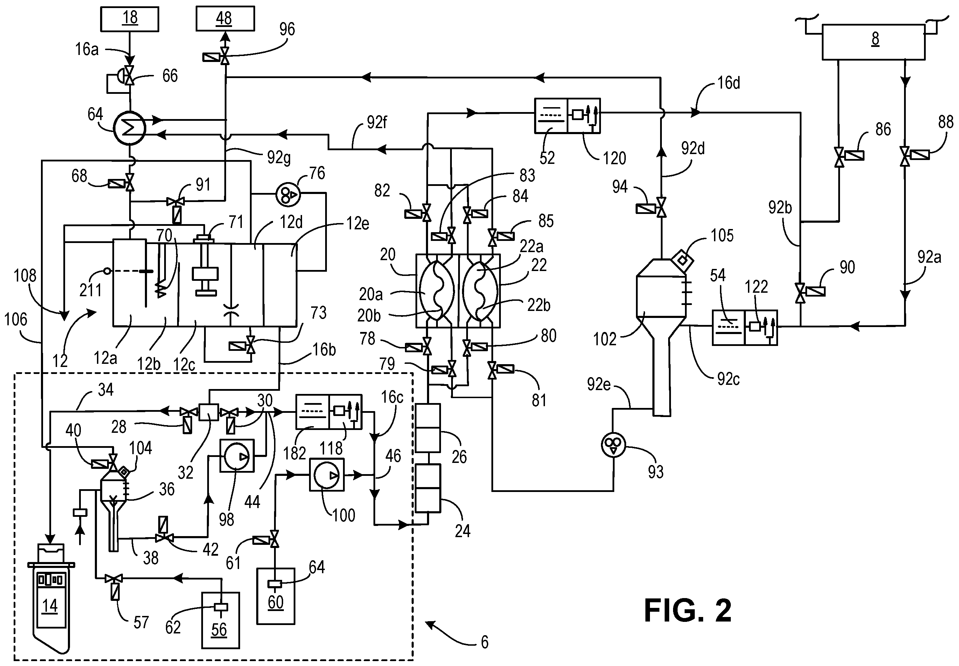

Referring to FIG. 1, a dialysis system 2 includes a dialysis machine 4 that includes a hydraulic system 5 (positioned mainly inside the dialysis machine 4 but schematically depicted in FIG. 2) to prepare dialysate solution and conduct the solution to and from a dialyzer 8. During treatment a disposable fluid line set 7 and the dialyzer 8 are connected to the dialysis machine 4 and to a patient 10. A peristaltic pump 11 circulates the patient's blood through the fluid line set 7 and the dialyzer 8. Fluid lines from the hydraulic system 5 also extend to the dialyzer 8 to allow the dialysate to pass through the dialyzer 8 alongside the blood. As the blood and dialysate pass through the dialyzer 8, toxins move across a semi-permeable surface of the dialyzer 8 from the blood to the dialysate. The dialysate is prepared with a concentration of salts from a container 14, an acid, and fresh water. The dialysate properties are typically tailored to physiology of the patient 10. Conductivity detectors 50, 52, 54 (shown in FIG. 2), which will be described in more depth below, monitor the concentration of dialysate throughout the hydraulic system 5 such that the dialysate can maintain a desired salt concentration. The conductivity detectors 50, 52, 54 are calibrated for the machine to correlate the measurements of the conductivity detectors 50, 52, 54 with the conductivity of the dialysate. A display 15 visualizes calibration-related information. By implementing an automated calibration method to calibrate the conductivity detectors 50, 52, 54, the setup process is much less user intensive. As a result, when used in a clinical setting, a user can calibrate several machines simultaneously with greater consistency and accuracy. The conductivity detectors 50, 52, 54 will further be more accurate in the range of conductivity of dialysate solution expected during treatment than conductivity detectors that are calibrated using a traditional one-point manual calibration technique.

FIG. 2 shows the hydraulic system 5 of the dialysis machine 4 during treatment. Many of the illustrated components are housed inside the machine 4 and are thus not visible in FIG. 1. By way of a general overview of the hydraulic system 5 during treatment, fluid (e.g. purified water) enters the dialysis machine 4 via a fluid source 18. The hydraulic system 5 includes mainline segments 16a-d (collectively referred to as a mainline 16) that are fluidly coupled to chambers 12a-e (collectively referred to as a hydrochamber 12). Fluid exits the hydrochamber 12 and enters a subsystem 6 of the hydraulic system 5. The subsystem 6 prepares a dialysate solution from the fluid, a salt concentrate (e.g., a buffer solution), and an acid. The subsystem 6 further includes the conductivity detector 50, which is used to monitor the conductivity of a bicarbonate solution within the subsystem 6 to help ensure that the dialysate exiting the subsystem 6 has a desired bicarbonate concentration. Following the subsystem 6, the fresh dialysate is conducted through mixing chambers 24, 26 and balancing chambers 20, 22. Fresh dialysate passes through a conductivity detector 52 and enters the dialyzer 8 via the mainline 16. The conductivity detector 52 is used to ensure that the dialysate being delivered to the dialyzer 7 has a desired concentration of 20 meq bicarbonate per liter to 40 meq bicarbonate per liter. The dialysate passes through the dialyzer 8 along with a patient's blood to filter the blood and remove toxins from the blood. The machine 4 further includes return line segments 92a-g (collective referred to as a return line 92). Spent dialysate containing the removed toxins exits the dialyzer via the return line 92, along which the conductivity detector 54 is located, and enters the balancing chambers 20, 22. The conductivity detector 54 measures the conductivity of spent dialysate as it exits the dialyzer 8 to determine the effectiveness of dialysis treatment. The spent dialysate is sent from the balance chamber 20, 22 to the drain 48.

The hydraulic system 5 will now be described in greater detail. The fluid source 18 can provide any appropriate type of fluid or fluids, such as a reverse osmosis water (RO water). Fluid from the fluid source 18 flows through the mainline segment 16a to the hydrochamber 12. A heat exchanger 64, a pressure regulator valve 66, and a control valve 68 are provided along the mainline segment 16a between the fluid source 22 and the hydrochamber 12. The heat exchanger 64 transfers heat from spent dialysate heading toward the drain 48 to the fluid from the fluid source 18. The pressure regulator valve 66 is put into the closed configuration when the fluid pressure exceeds safe levels, and a shut-off valve 88 controls flow into the hydrochamber 12.

The hydrochamber 12 is a multichambered unit (chambers 12a-12e being illustrated) that serves several functions. The fluid temperature within the hydrochamber 12 is monitored and/or controlled by a control thermostat 74 disposed in the chamber 12a. A heater 70 in the chamber 12b heats the fluid based on the temperature measured by the control thermostat 74. In chamber 12c, air is vented to a venting structure 71 as the fluid flows through the various chambers 12a-e of the hydrochamber 12. A deaeration pump 76 pumps fluid between the chamber 12d and the chamber 12e to return the fluid to the mainline segment 16b.

To assist in the separation of gases from the fluid contained within the hydrochamber 12, the chamber 12c of the hydrochamber 12 includes the venting structure 71. Gases entering the chamber 12d from a degassing line 106 can go through a bypass valve 73 to reach the third chamber 12c, rise upward through the fluid contained within the hydrochamber 12 to the upper portion of the chamber 12c to be vented through the venting structure 71 to the atmosphere 108. The bypass valve 73 is typically a shut-off valve. Under normal operation, only gases are typically vented through the degassing line 106 to the hydrochamber 12. During cleaning modes of the machine, the valve 73 is opened in order to relieve pressure built up within the hydrochamber 12.

Leaving the hydrochamber 12, fluid enters the mainline segment 16b and flows into the subsystem 6 of the hydraulic system 5. The subsystem 6 of the hydraulic system 5 prepares the dialysate solution, the process of which is discussed in further detail below. The subsystem 6 also includes the conductivity detector 50, which measures the conductivity of a salt and fluid solution formed within the subsystem 6. Dialysate leaves the subsystem 6 as dialysate and is directed to mixing chambers 24 and 26, which mixes the dialysate to create a substantially homogeneous solution.

The dialysate continues to the balancing chambers 20, 22, through which flow is controlled by shut-off valves 78-85. Each of the balancing chambers 20, 22 includes two separate subchambers 20a-b and 22a-b, respectively. Subchambers 20a and 20b and subchambers 22a and 22b are each separated by flexible membranes.

Within the balancing chambers 20, 22, fresh dialysate from the subsystem 6 passes into one or both of subchambers 20a, 22a through the valves 78 and 80, respectively. The fresh dialysate fills the subchambers 20a, 22a, causing the flexible membranes to move into the adjacent subchamber 20b, 22b. As a result, spent dialysate, which was previously delivered to the subchamber 20b, 22b from the dialyzer 8, is expelled from the subchambers 20b, 22b. Subsequently, additional spent dialysate from the dialyzer 8 is pumped into the subchambers 20b, 22b, causing fresh dialysate to be expelled from the subchambers 20a, 22a and flow to the dialyzer 8. This process results in a balanced provision of fresh dialysate and spent dialysate from and to, respectively, the dialyzer 8 during use.

Leaving the subchambers 20a and 22a through the valves 82 and 84, respectively, the fresh dialysate is directed through the mainline segment 16d. The conductivity detector 52, which will be described in more detail later, measures the conductivity of the fresh dialysate flowing through the mainline segment 16d. A shut-off valve 86 in the mainline segment 16d and a bypass shut-off valve 90 in a bypass line 92b control fresh dialysate flow into the dialyzer 8. Dialysate flowing through the mainline segment 16d moves on to the dialyzer 8 when the valve 86 is in the open configuration and the bypass valve 90 is in the closed position. When the valve 86 is in the closed position and the bypass valve 90 is in the open position, fluid passes through the bypass line 92b. The fresh dialysate can, for example, be diverted through the bypass line 92b when the conductivity detector 52 detects a conductivity that is outside of an acceptable interval. This prevents incorrectly formulated dialysate from contacting the patient's blood within the dialyzer 8.

During treatment, both fresh dialysate and patient blood flows into the dialyzer 8. As a result, toxins, such as urea, are transferred across a semi-permeable structure (e.g., semi-permeable microtubes) of the dialyzer 8 from the patient's blood to the dialysate.

Following the dialyzer 8, spent dialysate (i.e. dialysate containing toxins) exits the dialyzer 8 and passes a shut-off valve 88 to return to the hydraulic system 5. The valve 88 controls spent dialysate flow out of the dialyzer into the return line segment 92a. When the control valve 88 is in the open position and the bypass valve 90 is in the closed position, spent dialysate flows into the return line segment 92c. The conductivity detector 54, which will be described in more detail later, measures the conductivity of the dialysate flowing through the return line segment 92c. The measurements of the conductivity detector 54 can, for example, be used in conjunction with the measurements of the conductivity detector 52 to determine the sodium concentration of the patient's blood and/or a clearance value associated with the treatment.

Spent dialysate passes into an air separation chamber 102 before reaching the balancing chambers 20, 22 to ensure accurate operation of the balancing chambers 20, 22. An air sensor 105 is provided in air separation chamber 102 to provide an indication of when a shut-off valve 94 should be opened to allow passage of gases from the air separation chamber 102 to the return line segment 92d. From the air separation chamber 102, separated gases, and potentially fluid, are passed through the return line segment 92d to the drain 48 by opening the valves 94 and 96. The air sensor 105 is a two-pronged air detection probe located at the top of the air separation chamber 102 such that an electric current between the two prongs is detected when dialysate fills the air separation chamber 102 to at least the level of the prongs. Conversely, when there is air in the air separation chamber 102, the air between the two prongs of the air sensor 105 acts as an insulator and electric current does not flow.

Spent dialysate, from which the gases have been separated in the air separation chamber 102, is pumped by a dialysate pump 93 through return line segment 92e to one or both of the subchambers 20b, 22b through the valves 79, 81, respectively. The dialysate pump 93 is a step pump. As described above, this causes fresh dialysate to be expelled from the subchambers 20a, 22a. Fresh dialysate is subsequently pumped into the subchambers 20a, 22a to expel the spent dialysate from the subchambers 20b, 22b. Leaving one or both of the subchambers 20b, 22b through the valves 83, 85, respectively, the spent dialysate flows through the return line 92f and is directed to the heat exchanger 64, where the spent dialysate typically transfers heat originally received from the patient blood when it pass through the dialyzer to the fresh dialysate. A bypass shut-off valve 91 and the valve 96 control the flow of fluid into the drain 48. When the bypass valve 91 is closed and the valve 96 is open, spent dialysate flows the valve 96 into the drain 48.

The structure and operation of the subsystem 6 for preparation of the salt solution will now be explained in greater detail. Still referring to FIG. 2, the fluid line 16b bifurcates at a junction 32. A shut-off valve 28 and a shut-off valve 30 control the flow of fluid (e.g. RO water) to the mainline segment 16c and a subsystem line 34. If the valve 28 is closed and the valve 30 open, the fluid continues through the valve 30 to the mainline segment 16c. Conversely, if the valve 28 is open and the valve 30 closed, fluid proceeds through the valve 28 to the subsystem line 34.

During treatment, fluid flowing through the mainline segment 16b from the hydroblock 12 is directed to the subsystem 6 by opening the valve 28 and closing the valve 30 to provide flow to the subsystem line 34. To prepare a salt solution during treatment, fluid from the subsystem line 34 enters the container 14, which contains a powdered salt concentrate. The container 14 is a collapsible, replaceable bag that encloses the powdered salt concentrate. The subsystem 6 further includes a salt solution port with a salt solution filter 62 that can be connected to a salt solution source 56. During calibration, the salt solution source 56 can be used as an alternative or in addition to the container 14. A shut-off valve 57 controls flow from the salt solution source 56. During treatment, the valve 57 is typically closed because the salt solution source 56 is not typically used.

In order to expel air from the subsystem 6, an air separation chamber 36 is provided within the subsystem 6. In order to determine when gas has accumulated in the air separation chamber 36, an air sensor 104 is provided in the air separation chamber 36. The air separation chamber 36, which is fluidly connected to the container 14 by the subsystem line 34, is designed to remove both gases residually disposed within the container 14 and gases precipitating out of the salt solution during operation of the subsystem 6. During operation, air rises to the top of the air separation chamber 36, while the salt solution settles to the bottom of the air separation chamber 36. Salt solution is passed from the air separation chamber 36 to subsystem line 38, while gases are passed from the air separation chamber 36 to the degassing line 106 by operation of a shut-off valve 40. The air sensor 104 is a two-pronged air detection probe located at the top of the air separation chamber 36 such that an electric current between the two prongs is detected when dialysate fills the air separation chamber 36 to at least the level of the prongs. Conversely, when there is air in the air separation chamber 36, the air between the two prongs of the air sensor 104 acts as an insulator and electric current does not flow.

Air flow through the air separation chamber 36 is controlled by the valve 40. If the air sensor 104 does not detect air in the air separation chamber 36, the valve 40 is closed, and the solution proceeds through subsystem line 38, advanced by a salt solution pump 98 to rejoin the mainline segment 16c at a junction 44. The salt solution pump 98 is a step pump. Conversely, if the air sensor 104 detects air in the air separation chamber 36, the valve 40 is opened to vent gases from the air separation chamber 36 to the degassing line 106. The degassing line 106 provides a fluid connection to the hydrochamber 12 such that gases accumulated in the air separation chamber 36 are passed to the hydrochamber 12. The degassing line 106 is connected to the chamber 12d of the hydrochamber 12. In use, only gases, rather than an air/salt combination, typically are released from the separation chamber 36 through the valve 40, which is typically open for very short periods of time.

Turning first to the passage of salt solution from the air separation chamber 36, flow through the subsystem line 38 is controlled by operation of a shut-off valve 42. When the valve 42 is in the open position and the valve 40 is in the closed position, the salt solution flows through subsystem line 38 to the salt solution pump 98. The salt solution pump 98 pumps the salt solution to rejoin the mainline segment 16c at the junction 44. The salt solution passes through the conductivity detector 50 as it is conducted through the mainline segment 16c.

The subsystem 6 further includes an acid port with an acid filter 64 that can be connected to an acid solution container 60. A shut-off valve 61 controls flow from the acid solution container 60. When the valve 61 is open, an acid solution pump 100 pumps an acid solution that rejoins the mainline segment 16c at junction 46. The acid solution pump 100 is a step pump. The solution of fluid, salt, and acid then exits the subsystem 6 as dialysate and is directed toward the mixing chambers 24 and 26, then to one or both of the balancing chambers 20, 22, and on to the dialyzer 8 through the mainline segment 16d, as explained above.

As described above, the conductivity of the dialysate is measured at several points via the conductivity detectors 50, 52, 54. Disposed between junction 44 and junction 46, the conductivity detector 50 measures a value dependent on the conductivity of the mixture of fluid and salt solution. Disposed after the dialysate exits the balancing chamber 20, 22, conductivity detector 52 measures a value dependent on the conductivity of the solution of fluid, bicarbonate, and acid flowing into the dialyzer 8. Disposed after spent dialysate exits the dialyzer, conductivity detector 54 measures a value dependent on the conductivity of spent dialysate flowing from the dialyzer 8.

The conductivity detectors 50, 52, 54 are configured to measure conductivity of solutions at a specific temperature (e.g., 25 degrees Celsius). The temperature of the solution, however, varies as it is being raised to be passed through the dialyzer 8 to the patient. Therefore, the conductivity of the dialysate is determined by adjusting the signal received from each conductivity detector 50, 52, 54 based on the temperature of the solution as measured by temperature detectors 118, 120, 122 respectively. The amounts of adjustment are determined using equations that approximate temperature dependence of conductivity, such as the Steinhart-Hart equation. The temperature detectors 118, 120, 122 are typically thermistors, which are resistors in which the resistance varies significantly with temperature.

### Methods of Use

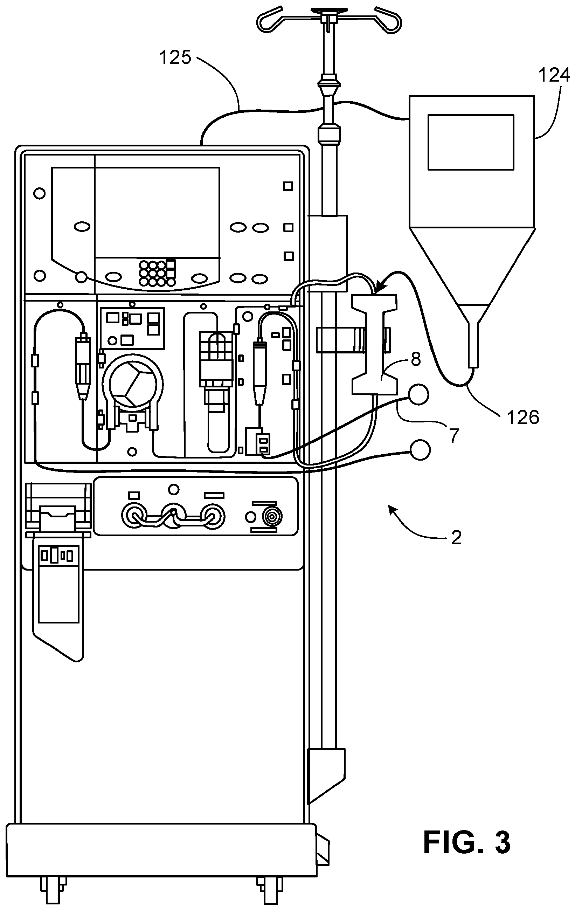

FIG. 3 shows the dialysis system 2 during a process used to calibrate the conductivity detectors 50, 52, 54. A conductivity meter 124 with a connection cable 125 and a calibration meter probe 126 is used to take a conductivity measurement of dialysate flowing into the dialyzer 8. The connection cable 125 is connected to the dialysis system 2 via an analog input port located on the rear of the dialysis machine 4. The calibration meter probe 126 is put in fluid communication with the dialyzer 8. The patient lines of the disposable fluid line set 7 are disconnected from the patients during this process. Referring back to FIG. 2, during calibration, the container 14 is disconnected, the valve 28 is closed, and the valve 30 is opened. The valve 57 is further placed into the open position so that the salt solution pump 98 conducts salt solution from the salt solution source 56 into the mainline segment 16c. During the calibration process, the salt solution source 56 is used in lieu of the container 14 so that the salt solution combined with the fluid in the subsystem 6 has a constant sodium concentration. The valve 61 is also placed into the open position so that the acid solution pump 100 conducts acid solution from the acid solution container 60 into the mainline segment 16c.

The dialysis system 2 further includes a controller (not shown), such as a Microchip PIC18F6410 (manufactured by Microchip Technology, Inc. (Chandler, Arizona)), which is capable of receiving signals from and activating one or more of the pumps 93, 98, 100 and one or more of the valves 28, 30, 40, 42, 57, 61, 66, 73, 78-86, 88, 90-91, 94, 96; receiving input from air sensors 104, 105 and conductivity detectors 50, 52, 54; and receiving input from an external device electrically connected to the system.

The controller further receives from the conductivity meter 124 via the connection cable 125 a time series of conductivity meter measurement of dialysate passing through the dialyzer. The controller also receives a time series of sensor measurements from each of the conductivity detectors 50, 52, 54.

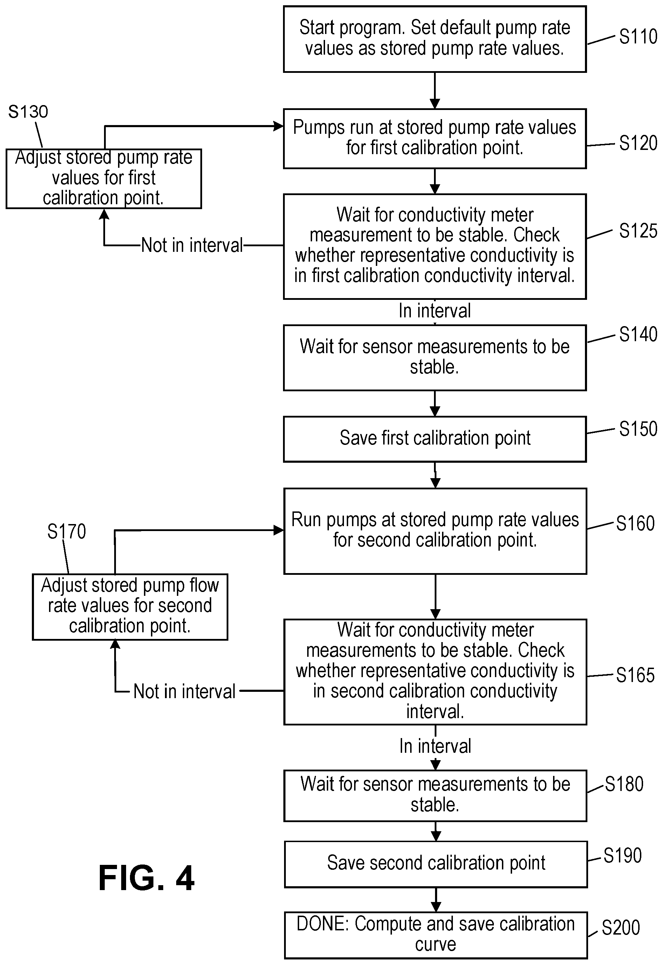

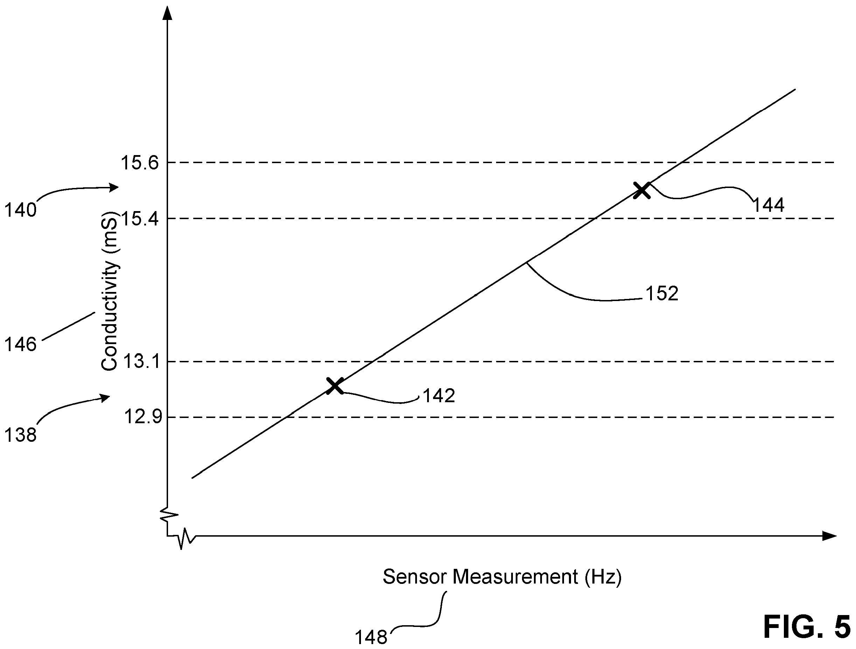

FIG. 4 shows an exemplary flow chart of a program to calibrate a conductivity detector (e.g. conductivity detectors 50, 52, 54 of FIG.2). FIG. 5 shows an exemplary calibration curve 152 for the conductivity detector that is computed after the controller executes the program of FIG. 4. This calibration process can, for example, be carried out during manufacturing or shortly after manufacturing of the dialysis machine. By way of general overview, referring to FIG. 4, the controller is loaded with a calibration program 200 with steps S110, S120, S125, S130, S140, S150, S160, S165, S170, S180, S190, and S200. The controller executes this program to simultaneously calibrate the conductivity detectors (e.g. conductivity detectors 50, 52, 54 of FIG. 2). The calibration program 200 allows the controller to calibrate the conductivity detector to two calibration points 142, 144. Each calibration point 142, 144 is associated with a representative sensor measurement 148 and a representative conductivity 146, both of which will be explained in further detail below. The calibration points 142, 144 reflect the conductivities of the dialysate typically used during treatment. For example, the two calibration points 142, 144 can correspond to approximate minimum and maximum conductivities expected during treatment of an average human patient.

Prior to starting the program, default fluid flow rates for the pumps for each calibration point 142, 144 are loaded into a memory storage element connected to the controller.

In step S 110, the controller starts the program and sets the stored pump rate values for both the first and second calibration points to be the default pump rate values. The controller receives sensor measurements from the conductivity detectors 50, 52, 54 and receives conductivity meter measurements from the conductivity meter 128, which measures the conductivity of the fluid entering the dialyzer inlet.

The system first calibrates the conductivity detector to the first calibration point 142. In step S120, the controller runs the salt solution pump 98, the acid solution pump 100, and the dialysate pump 93 (shown in FIG. 2) at their stored flow rate values for the first calibration point 142.

In step S125, the controller waits for the conductivity meter measurement to reach a stable value. Stability of the conductivity meter measurements is determined by a duration of time that the measurements are between two values. For example, in a period of 500 ms, conductivity meter measurements that stay within a range of 0.1 mS/cm can indicate stability. When the controller determines that the conductivity meter measurements are stable, the controller then computes a representative conductivity 146 from the conductivity meter measurements. The representative conductivity 146 is an average of the conductivity meter measurements over a duration of time (i.e. a moving average). A first calibration conductivity interval 138 for the first calibration point 142 is set to reflect a lower bound for typical human blood conductivity ranges. The first conductivity calibration interval 138 can be, for example, between 12 mS/cm and 14 mS/cm. The controller compares the representative conductivity 146 to the first calibration conductivity interval 138.

If the representative conductivity 146 is not within the first calibration conductivity interval 138, the controller proceeds to step S130 and adjusts the flow rates of the salt solution, the acid solution, and the dialysate pumps to increase or decrease the representative conductivity 146 toward the first calibration conductivity interval 138. Adjustments to the pump flow rates are a function of the difference between the first calibration interval 138 and the representative conductivity 146. For example, the magnitude of an increase or decrease in the pump flow rate can be directly proportional to the difference between the first calibration interval 138 and the representative conductivity 146. The controller continues to implement steps S120, S125, and S130 until the representative conductivity 146 falls within the first calibration conductivity interval 138.

Once the representative conductivity 146 is within the first calibration conductivity interval 138, the controller proceeds to step S140 and waits until the sensor measurements are stable. Stability of the sensor measurements is determined by a duration of time that the measurements are between two values. For example, in a period of 500 ms, sensor measurements that stay within a range of 10 Hz can indicate stability. When the controller determines that the sensor measurements are stable, the controller then computes a representative sensor measurement 148 from the sensor measurements.

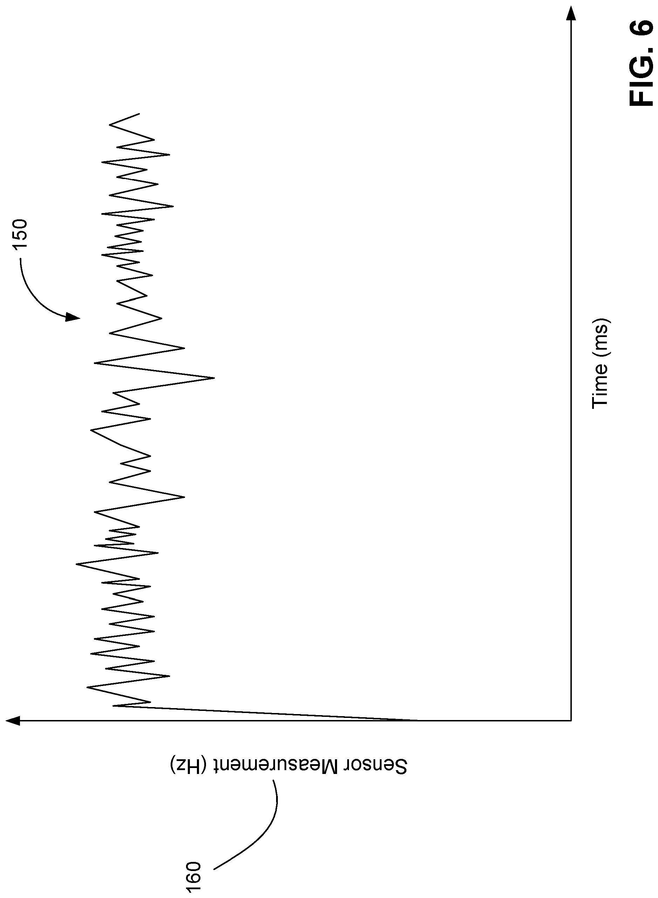

Referring to FIG. 6, sensor measurements 160 in step S140 can express a transient response 150 that can have a large range of values at steady state. For example, in the hydraulic arrangement as shown in FIG. 2, the conductivity detector 50 is disposed prior to the fluid reaching the mixing chambers 24, 26, which can large variability in the sensor measurements 160. As a result, stability of a single sample of sensor measurements can be difficult to achieve. To reduce the variability caused by the transient response, the representative sensor measurement 148 (shown in FIG. 5) is a moving average of a time series of sensor measurements, and the representative sensor measurement 148 is further used to determine stability of the sensor measurements. The controller determines that the sensor measurements are stable if the representative sensor measurement 148 is within a range of 10 Hz. The moving average is for a duration of time (e.g., 500 ms to 1000 ms) appropriate for producing a stable representative sensor measurement 148 (shown in FIG. 5). In some cases, the conductivity is defined as stable if a maximum absolute conductivity difference between several consecutive samples (e.g., 5 to 15 samples, 15 to 30 samples, 30 to 45 samples) of the representative sensor measure 148 is within a predefined range (e.g., less than 0.02 mS/cm, 0.05 mS/cm, or 0.1 mS/cm, or between 0 and 0.02 mS/cm, 0 and 0.05 mS/cm, or 0 and 0.1 mS/cm).

Once the controller determines that the sensor measurements are stable, in step S150, the controller saves the representative conductivity 146 and computes and saves a representative sensor measurement 148 from sensor measurements of the conductivity detector. The controller sets the saved representative sensor measurement 148 and the saved representative conductivity 146 to correspond to the first calibration point 142.

Still referring to FIG. 4, in step S160, the controller runs the salt solution pump 98, the acid solution pump 100, and the dialysate pump 93 (shown in FIG. 2) at their stored flow rate values for the second calibration point 144.

In step S165, the controller waits for the conductivity meter measurement to reach a stable value using the method as described above in step S125 for the first calibration point. When the controller determines that the conductivity meter measurements are stable, the controller then computes a representative conductivity 146 from the conductivity meter measurements. A second calibration conductivity interval 140 is set to reflect an upper bound for typical human blood conductivity ranges. The second conductivity calibration interval 138 can, for example, be between 14mSiemens/cm and 16 mS/cm. The controller compares the representative conductivity 146 to the second calibration conductivity interval 140.

If the representative conductivity 146 is not within the second calibration conductivity interval 140, the controller proceeds to step S170 and adjusts the pump rates of the salt solution pump, the acid solution pump, and the dialysate pump to increase or decrease the representative conductivity 146 toward the second calibration conductivity interval 140. Increasing the pump rates of the salt solution pump and the acid solution pump increase the conductivity of the fluid circulating through the fluid circuit. As described above, the magnitude of an increase or decrease in the pump flow rate is directly proportional to the difference between the second calibration interval 140 and the representative conductivity 146. The controller continues to implement steps S160, S165, and S170 until the representative conductivity 146 falls within the second calibration conductivity interval 140.

Once the representative conductivity 146 is within the second calibration conductivity interval 140, the controller proceeds to step S180 and waits until the sensor measurements are stable using the method as described above in step S140 for the first calibration point.

Once the controller determines that the sensor measurements are stable, in step S190, the controller saves the representative conductivity 146 and computes and saves a representative sensor measurement 148 from sensor measurements of the conductivity detector. The controller sets the saved representative sensor measurement 148 and the representative conductivity 146 to correspond to the second calibration point 144 of the conductivity detector.

In step S200, the controller completes calibration by computing the calibration curve 152 for the conductivity detector. The calibration curve relates the sensor measurements of the conductivity detector and the conductivity of the fluid passing the conductivity detector. Referring to FIG. 5, the example calibration curve 152 includes the first and second calibration points 142, 144 derived from the two-point calibration described above. The calibration curve 152 can be used to determine an estimated conductivity from a sensor measurement. The first calibration point 142 and the second calibration point 144 define the conductivity curve 152, which consists of a linear interpolation and extrapolation from the two calibration points 142, 144. In the example illustrated in FIG. 5, the first calibration point 142 lies within the first calibration conductivity interval 138 set between 12.9 mS/cm and 13.1 mS/cm. Still referring to FIG. 5, the second calibration point 142 lies within the second calibration conductivity interval 140 set between 15.4 mS/cm and 15.6 mS/cm.

While the bypass valves 73, 90, 91 have been described as being closed during treatment, in some implementations, the bypass valves 73, 90, 91 may be placed into the open position for other functions. For example, the bypass valves 73, 90, 91 can be placed into the open position to clean the hydraulic system 5. In cooperation with closing other valves in the hydraulic system 5, the bypass valves 73, 90, 91 can also be opened in response to unsafe pressures for treatment so that the patient is not exposed to high fluid pressure. For example, when the valve 88 is in the closed position, the control valve 86 is in the closed position, and the bypass valve 90 is in the open position, fluid from the mainline 16d flows into the return line segment 92c without passing into the dialyzer 8.

While the valves 28, 30, 40, 42, 57, 61, 66, 73, 78-86, 88, 90-91, 94, 96 in the hydraulic system have been described as shut-off valves, in other implementations, the valves may be multi-position valves. For example, the valves 28, 30 may be replaced by a single valve that includes three positions: one position that arrests flow entirely, one position that directs flow to the subsystem line 34, and one position that directs flow along the mainline segment 16c.

While the container 14 of salt is described as a collapsible, replaceable bag that encloses the powdered salt concentrate, in other implementations, the container can be a rigid container that holds a solution of unknown salt concentration.

In some implementations, the salt solution source 56 is a jug of a salt solution, such as sodium bicarbonate concentration. While the salt solution source 56 has been described as different from the container 14, the salt concentrate from the container 14 can be used during calibration.

While the dialyzer 8 has been described as being connected to the fluid circuit during calibration, in other implementations, the dialyzer can be removed and the portion of the fluid circuit connected to the inlet of the dialyzer can be directly connected to the portion of the fluid circuit connected to the outlet of the dialyzer.

While the temperature detectors 118, 120, 122 have been described as thermistors, in other implementations, the temperature detectors are other temperature sensors of the art. For example, the temperature detectors can be thermocouples, where one end is placed in contact with the solution and the other end is placed in a reference solution or environment.

While the conductivity detectors 50, 52, 54 and the temperature detectors 118, 120, 122 have been described as separate sensors, in some implementations, a single sensor unit can comprise a conductivity sensor to measure the conductivity and a thermistor to measure the temperature.

In some implementations, the conductivity detectors 50, 52, 54 can be replaced by other types of sensors configured to measure the concentration of a solution in fluid communication with it. For example, an optical or ultrasonic sensor can be used to detect the amount of dissolved salt in the solution. The amount of transmitted light or ultrasound can be correlated with the concentration of the solution.

While the conductivity meter probe 126 has been described as being in fluid communication with the dialyzer inlet, in other implementations, the conductivity meter probe can be placed in fluid communication with the dialyzer outlet. Alternatively, the conductivity meter probe 126 could be in fluid contact to the dialysate line at any of various other points. For example, the conductivity meter probe 126 could be put in fluid communication with the mainline segment 16c after the temperature detector 120.

While the controller has been described to execute the program to calibrate simultaneously the conductivity detectors 50, 52, 54 of the hydraulic system 5, in some implementations, the controller can calibrate the conductivity detectors one-by-one.

While the controller has been described to adjust the pump rate of the pumps 93, 98, 100 in the hydraulic system 5 to adjust the conductivity of the solution, in some implementations, the controller can adjust the conductivity by adjusting valves in the hydraulic system. For example, the controller can adjust the valves to increase or decrease the flow rate of the individual streams of solutions (e.g., the acid solution, the salt solution, or other buffer solutions) and therefore increase or decrease the conductivity of the solution.

While the conductivity calibration intervals 138, 140 have been described as having an upper and lower threshold, in some implementations, a calibration conductivity range (e.g., calibration conductivity ranges 138 and 140) can be defined by a target conductivity that exhibits hysteresis. For example, the target conductivity could be 13 mS/cm with symmetric bidirectional limits of ±1 mS/cm.

While two calibration points 142, 144 have been described, in other implementations, the conductivity curve can be defined by a single calibration point and the origin. The conductivity curve need not be limited to a linear interpolation and extrapolation and, in some implementations, is computed through a polynomial or non-polynomial extrapolation and interpolation. In some implementations, the conductivity curve can be defined by more than two calibration points. For example, for a third degree polynomial fit, the calibration curve is defined by three calibration points.

While the calibration has been described to apply to the three conductivity detectors 50, 52, 54, fewer or more conductivity detectors can be calibrated within the system.

While the pump adjustments in steps S130 and S170 of the calibration method have been described to be directly proportional to the difference between the representative conductivity and the calibration interval, in other implementations, the pump adjustments can be fixed or can have a nonlinear relationship with the difference. For example, an integral or derivative gain can be added to decrease overshoot and/or decrease rise time.

While, after making pump adjustments in steps S130 and S170, the controller has been described to compute the representative conductivity in steps S125 and S165, respectively, in some implementations, the controller includes a delay between the pump adjustments and the computation of the representative conductivity. For example, the delay can be between, for example, 10 seconds to 20 seconds, 20 seconds to 30 seconds, 30 seconds to 40 seconds to provide sufficient time for the conductivity of the fluid circulating in the fluid circuit to change due to the pump adjustments. In some cases, the delay can be greater than 30 seconds, 40 seconds, or 50 seconds.

While the calibration method has been described to apply to conductivity detectors 50, 52, 54, the controller can be configured such that the calibration method could work for other types of detectors as well, not limited to flow rate sensors, temperature sensors, and pressure sensors. For example an external calibrated temperature meter or thermometer can be placed in fluid communication with the hydraulic system and electrical communication with the controller. The controller can execute a calibration program of the temperature sensors in the hydraulic system.

While the controller has been described to begin taking conductivity meter measurements and sensor measurements from the conductivity meter 128 and the conductivity detectors 50, 52, 54 at step S110, in some implementations, an algorithm can be implemented such that the controller takes measurements at a shorter time interval after the measurements have exceeded a threshold to reduce the processing burden on the controller. For example, at S110, the controller can take measurements from the conductivity meter every 100 ms. When the conductivity meter measurement exceeds 8 mS/cm, the controller begins taking measurements from the conductivity meter every 10 ms.

While the duration of time for the moving average of sensor measurement that is used to compute the representative sensor measurement 148 has been described to be 500 ms, in some implementations, this duration of time can be between 200 and 2000 ms.

While the calibration method has been described to apply a moving average to the conductivity meter measurements and sensor measurements, in other implementations, the calibration method can use a weighted moving average, a moving median, or other smoothing methods known in the art.

While the controller has been described to adjust pump rates of the dialysate pump 93, the acid solution pump 100, and the salt solution pump 98 to achieve a target conductivity, in other implementations, the controller can adjust just one or two of the pumps. For example, adjusting only the salt solution pump can induce a change in conductivity for all three downstream conductivity detectors.

While the system 2 has been described as a hemodialysis system, the calibration methods described herein can be applied in other types of medical systems, including peritoneal dialysis systems, apheresis systems, infusion pumps systems, etc.

While only one controller is described, multiple controllers may alternatively be used. Implementations of the subject matter and the operations described in this specification can be implemented in digital electronic circuitry, or in computer software, firmware, or hardware, including the structures disclosed in this specification and their structural equivalents, or in combinations of one or more of them. Implementations of the subject matter described in this specification can be implemented as one or more computer programs, i.e., one or more modules of computer program instructions, encoded on computer storage medium for execution by, or to control the operation of, data processing apparatus. Alternatively or in addition, the program instructions can be encoded on an artificially generated propagated signal, for example, a machine-generated electrical, optical, or electromagnetic signal, that is generated to encode information for transmission to suitable receiver apparatus for execution by a data processing apparatus. A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially generated propagated signal. The computer storage medium can also be, or be included in, one or more separate physical components or media (for example, multiple CDs, disks, or other storage devices).

The operations described in this specification can be implemented as operations performed by a data processing apparatus on data stored on one or more computer-readable storage devices or received from other sources.

The term "data processing apparatus" encompasses all kinds of apparatus, devices, and machines for processing data, including by way of example a programmable processor, a computer, a system on a chip, or multiple ones, or combinations, of the foregoing. The apparatus can include special purpose logic circuitry, for example, an FPGA (field programmable gate array) or an ASIC (application specific integrated circuit). The apparatus can also include, in addition to hardware, code that creates an execution environment for the computer program in question, for example, code that constitutes processor firmware, a protocol stack, a database management system, an operating system, a cross-platform runtime environment, a virtual machine, or a combination of one or more of them. The apparatus and execution environment can realize various different computing model infrastructures, such as web services, distributed computing and grid computing infrastructures.

A computer program (also known as a program, software, software application, script, or code) can be written in any form of programming language, including compiled or interpreted languages, declarative or procedural languages, and it can be deployed in any form, including as a standalone program or as a module, component, subroutine, object, or other unit suitable for use in a computing environment. A computer program may, but need not, correspond to a file in a file system. A program can be stored in a portion of a file that holds other programs or data (for example, one or more scripts stored in a markup language document), in a single file dedicated to the program in question, or in multiple coordinated files (for example, files that store one or more modules, sub programs, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform actions by operating on input data and generating output. The processes and logic flows can also be performed by, and apparatus can also be implemented as, special purpose logic circuitry, for example, an FPGA (field programmable gate array) or an ASIC (application specific integrated circuit).

Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing actions in accordance with instructions and one or more memory devices for storing instructions and data. Generally, a computer will also include, or be operatively coupled to receive data from or transfer data to, or both, one or more mass storage devices for storing data, for example, magnetic, magneto optical disks, or optical disks. However, a computer need not have such devices. Moreover, a computer can be embedded in another device, for example, a mobile telephone, a personal digital assistant (PDA), a mobile audio or video player, a game console, a Global Positioning System (GPS) receiver, or a portable storage device (for example, a universal serial bus (USB) flash drive), to name just a few. Devices suitable for storing computer program instructions and data include all forms of nonvolatile memory, media and memory devices, including by way of example semiconductor memory devices, for example, EPROM, EEPROM, and flash memory devices; magnetic disks, for example, internal hard disks or removable disks; magneto optical disks; and CD ROM and DVD-ROM disks. The processor and the memory can be supplemented by, or incorporated in, special purpose logic circuitry.

To provide for interaction with a user, implementations of the subject matter described in this specification can be implemented on a display device (e.g., the display device of the dialysis machine 12), for example, a CRT (cathode ray tube) or LCD (liquid crystal display) monitor, for displaying information to the user and a keyboard or keypad and/or a pointing device, for example, a mouse or a trackball, by which the user can provide input to the computer. Other kinds of devices can be used to provide for interaction with a user as well; for example, feedback provided to the user can be any form of sensory feedback, for example, visual feedback, auditory feedback, or tactile feedback; and input from the user can be received in any form, including acoustic, speech, or tactile input.

Other implementations are within the scope of the following claims

## Claims

1. A dialysis system (2) comprising:
at least one pump (93, 98, 100) disposed along a fluid circuit to conduct a fluid at a pump rate through the fluid circuit, wherein the pump rate is adjustable;
a conductivity meter comprising a probe (126) configured to be placed in fluid communication with the fluid circuit to measure conductivity values of the fluid;
at least one conductivity detector (50, 52, 54) disposed along the fluid circuit to measure sensor values of the fluid;
a controller configured to:
i) receive the conductivity values from the conductivity meter and the sensor values from the at least one conductivity detector;
ii) compute a plurality of representative conductivity values from the received conductivity values and a plurality of representative sensor values from the received sensor values;
iii) determine a first calibration point (142, 144) associated with a first representative conductivity value and a first representative sensor value;
iv) determine a second calibration point (142, 144) associated with a second representative conductivity value and a second representative sensor value; and
v) compute a calibration curve (152) based on the first and second calibration points.

2. The dialysis system of claim 1, wherein the controller is configured to adjust a pump rate of the at least one pump until the first representative conductivity value is within a calibration interval before the controller determines the first calibration point.

3. The dialysis system of claim 2, wherein the controller is configured to introduce a delay after the pump rate adjustment and before the first calibrationpoint determination, for example, wherein the delay is greater than 30 seconds.

4. The dialysis system of any one of the above claims, wherein the controller is configured to adjust a pump rate of the at least one pump until the second representative conductivity value is within a calibration interval (138, 140) before the controller determines the second calibration point, and optionally wherein the controller is configured to introduce a delay after the pump rate adjustment and before the second calibration point determination, for example, wherein the delay is greater than 30 seconds.

5. The dialysis system of any one of the above claims, wherein at least one of the first and second representative sensor values is a moving average of the received sensor values.

6. The dialysis system of any one of the above claims, further comprising a temperature sensor disposed along the fluid circuit, wherein the controller is configured to adjust the conductivity value of the at least one conductivity detector based on a temperature measured by the temperature sensor.

7. The dialysis system of any one of the above claims, wherein the controller is configured to determine a third calibration point, wherein the controller is configured to compute the calibration curve based on the first, second, and third calibration points, and optionally wherein the controller computes the calibration curve via a nonlinear polynomial fit of the first, second, and third calibration points.

8. A method for calibrating at least one conductivity detector disposed along a dialysis fluid circuit comprising:
i) passing a fluid through the dialysis fluid circuit by running at least one pump (93, 98, 100) disposed along the dialysis fluid circuit;
ii) receiving conductivity values from a conductivity meter (126) disposed along the dialysis fluid circuit and sensor values from the at least one conductivity detector (50, 52, 54) disposed along the dialysis fluid circuit;
iii) computing a plurality of representative conductivity values from the received conductivity values and a plurality of representative sensor values from the received sensor values;
iv) determining a first calibration point (142, 144) associated with a first representative conductivity value and a first representative sensor value;
v) determining a second calibration point (142, 144) associated with a second representative conductivity value and a second representative sensor value; and
vi) computing a calibration curve (152) based on the first and second calibration points.

9. The method of claim 8, further comprising, before determining the first calibration point, adjusting a pump rate of the at least one pump until the first representative conductivity value is within a calibration interval (138, 140).

10. The method of claim 9, further comprising introducing a delay after adjusting the pump rate and before determining the first calibration point, for example, wherein the delay is greater than 30 seconds.

11. The dialysis system of any one of claims 1-3 or the method of any one of claims 8-10, wherein the fluid is a solution of unknown concentration, for example, wherein the solution comprises salt and optionally further comprises an acid.

12. The method of any one of claims 8-11, wherein further comprising, before determining the second calibration point, adjusting a pump rate of the at least one pump until the second representative conductivity value is within a calibration interval, and optionally wherein the method further comprises introducing a delay after adjusting the pump rate and before determining the second calibration point, for example, wherein the delay is greater than 30 seconds.

13. The dialysis system of any one of claims 2, 3, or 4 or the method of any one of claims 9 or 12, wherein the calibration interval is defined by an upper threshold and a lower threshold.

14. The method of any one of claims 8-13, wherein at least one of the first and second representative conductivity values is a moving average of the received conductivity values.

15. The method of any one of claims 8-14, further comprising:
receiving temperature values from a temperature sensor disposed along the fluid circuit; and
adjusting the conductivity value of the at least one conductivity detector based on a temperature measured by the temperature sensor, and optionally wherein the conductivity detector and temperature sensor are a single sensor.

16. The method of any one of claims 8-15, further comprising determining a third calibration point, wherein computing the calibration curve is based on the first, second, and third calibration points, and optionally wherein computing the calibration curve further comprises a nonlinear polynomial fit of the first, second, and third calibration points.

17. The dialysis system of any one of claims 1 to 7 or the method of any one of claims 8-16, wherein the at least one conductivity detector comprises at least one of a), b), or c), wherein:
a) a conductivity detector disposed along the dialysis fluid circuit at a location upstream of a dialyzer;
b) a conductivity detector disposed along the dialysis fluid circuit at a location downstream of the dialyzer; and
c) a conductivity detector disposed along the dialysis fluid circuit in between a salt injection port in the dialysis fluid circuit and an acid solution injection port in the dialysis fluid circuit.

18. The dialysis system of any one of claims 1 to 7 or the method of any one of claims 8-16, wherein the fluid is a dialysate.

## Patentansprüche

1. Dialysesystem (2), umfassend:
mindestens eine Pumpe (93, 98, 100), die entlang eines Fluidkreislaufs angeordnet ist, um ein Fluid mit einer Pumprate durch den Fluidkreislauf zu leiten, wobei die Pumprate anpassbar ist;
ein Leitfähigkeitsmessgerät, das eine Sonde (126) umfasst, die ausgelegt ist, um in Fluidkommunikation mit dem Fluidkreislauf platziert zu werden, um Leitfähigkeitswerte des Fluids zu messen;
mindestens einen Leitfähigkeitsdetektor (50, 52, 54), der entlang des Fluidkreislaufs angeordnet ist, um Sensorwerte des Fluids zu messen;
eine Steuerung, die ausgelegt ist zum:
i) Empfangen der Leitfähigkeitswerte von dem Leitfähigkeitsmessgerät und der Sensorwerte von dem mindestens einen Leitfähigkeitsdetektor;
ii) Berechnen einer Vielzahl von repräsentativen Leitfähigkeitswerten aus den empfangenen Leitfähigkeitswerten und einer Vielzahl von repräsentativen Sensorwerten aus den empfangenen Sensorwerten;
iii) Bestimmen eines ersten Kalibrierungspunkts (142, 144), der einem ersten repräsentativen Leitfähigkeitswert und einem ersten repräsentativen Sensorwert zugeordnet ist;
iv) Bestimmen eines zweiten Kalibrierungspunkts (142, 144), der einem zweiten repräsentativen Leitfähigkeitswert und einem zweiten repräsentativen Sensorwert zugeordnet ist; und
v) Berechnen einer Kalibrierungskurve (152) basierend auf dem ersten und dem zweiten Kalibrierungspunkt.

2. Dialysesystem nach Anspruch 1, wobei die Steuerung ausgelegt ist, um eine Pumprate der mindestens einen Pumpe anzupassen, bis der erste repräsentative Leitfähigkeitswert innerhalb eines Kalibrierungsintervalls liegt, bevor die Steuerung den ersten Kalibrierungspunkt bestimmt.

3. Dialysesystem nach Anspruch 2, wobei die Steuerung ausgelegt ist, um nach der Pumpratenanpassung und vor der Bestimmung des ersten Kalibrierungspunkts eine Verzögerung einzubringen, wobei die Verzögerung beispielsweise größer als 30 Sekunden ist.

4. Dialysesystem nach einem der vorhergehenden Ansprüche, wobei die Steuerung ausgelegt ist, um eine Pumprate der mindestens einen Pumpe anzupassen, bis der zweite repräsentative Leitfähigkeitswert innerhalb eines Kalibrierungsintervalls (138, 140) liegt, bevor die Steuerung den zweiten Kalibrierungspunkt bestimmt, und wobei die Steuerung gegebenenfalls ausgelegt ist, um nach der Pumpratenanpassung und vor der Bestimmung des zweiten Kalibrierungspunkts eine Verzögerung einzubringen, wobei die Verzögerung beispielsweise größer als 30 Sekunden ist.

5. Dialysesystem nach einem der vorhergehenden Ansprüche, wobei mindestens einer von dem ersten und dem zweiten repräsentativen Sensorwert ein gleitender Durchschnitt der empfangenen Sensorwerte ist.

6. Dialysesystem nach einem der vorhergehenden Ansprüche, des Weiteren umfassend einen Temperatursensor, der entlang des Fluidkreislaufs angeordnet ist, wobei die Steuerung ausgelegt ist, um den Leitfähigkeitswert des mindestens einen Leitfähigkeitsdetektors basierend auf einer Temperatur anzupassen, die durch den Temperatursensor gemessen wurde.

7. Dialysesystem nach einem der vorhergehenden Ansprüche, wobei die Steuerung ausgelegt ist, um einen dritten Kalibrierungspunkt zu bestimmen, wobei die Steuerung ausgelegt ist, um die Kalibrierungskurve basierend auf dem ersten, zweiten und dritten Kalibrierungspunkt zu berechnen, und wobei die Steuerung gegebenenfalls die Kalibrierungskurve über eine nicht-lineare Polynomanpassung des ersten, zweiten und dritten Kalibrierungspunkts berechnet.

8. Verfahren zum Kalibrieren von mindestens einem Leitfähigkeitsdetektor, der entlang eines Dialysefluidkreislaufs angeordnet ist, umfassend:
i) Leiten eines Fluids durch den Dialysefluidkreislauf, indem mindestens eine Pumpe (93, 98, 100) laufen gelassen wird, die entlang des Dialysefluidkreislaufs angeordnet ist;
ii) Empfangen von Leitfähigkeitswerten von einem Leitfähigkeitsmessgerät (126), das entlang des Dialysefluidkreislaufs angeordnet ist, und Sensorwerten von dem mindestens einem Leitfähigkeitsdetektor (50, 52, 54), der entlang des Dialysefluidkreislaufs angeordnet ist;
iii) Berechnen einer Vielzahl von repräsentativen Leitfähigkeitswerten aus den empfangenen Leitfähigkeitswerten und einer Vielzahl von repräsentativen Sensorwerten aus den empfangenen Sensorwerten;
iv) Bestimmen eines ersten Kalibrierungspunkts (142, 144), der einem ersten repräsentativen Leitfähigkeitswert und einem ersten repräsentativen Sensorwert zugeordnet ist;
v) Bestimmen eines zweiten Kalibrierungspunkts (142, 144), der einem zweiten repräsentativen Leitfähigkeitswert und einem zweiten repräsentativen Sensorwert zugeordnet ist; und
vi) Berechnen einer Kalibrierungskurve (152) basierend auf dem ersten und dem zweiten Kalibrierungspunkt.

9. Verfahren nach Anspruch 8, des Weiteren umfassend Anpassen einer Pumprate der mindestens einen Pumpe vor dem Bestimmen des ersten Kalibrierungspunkts, bis der erste repräsentative Leitfähigkeitswert innerhalb eines Kalibrierungsintervalls (138, 140) liegt.

10. Verfahren nach Anspruch 9, des Weiteren umfassend Einbringen einer Verzögerung nach dem Anpassen der Pumprate und vor dem Bestimmen des ersten Kalibrierungspunkts, wobei die Verzögerung beispielsweise größer als 30 Sekunden ist.

11. Dialysesystem nach einem der Ansprüche 1 bis 3 oder Verfahren nach einem der Ansprüche 8 bis 10, wobei das Fluid eine Lösung mit unbekannter Konzentration ist, wobei die Lösung beispielsweise Salz und gegebenenfalls des Weiteren eine Säure umfasst.

12. Verfahren nach einem der Ansprüche 8 bis 11, des Weiteren umfassend Anpassen einer Pumprate der mindestens einen Pumpe vor dem Bestimmen des zweiten Kalibrierungspunkts, bis der zweite repräsentative Leitfähigkeitswert innerhalb eines Kalibrierungsintervalls liegt, und wobei gegebenenfalls das Verfahren des Weiteren Einbringen einer Verzögerung nach dem Anpassen der Pumprate und vor dem Bestimmen des zweiten Kalibrierungspunkts umfasst, wobei die Verzögerung beispielsweise größer als 30 Sekunden ist.

13. Dialysesystem nach einem der Ansprüche 2, 3 oder 4 oder Verfahren nach einem der Ansprüche 9 oder 12, wobei das Kalibrierungsintervall durch einen oberen Schwellenwert und einen unteren Schwellenwert definiert ist.

14. Verfahren nach einem der Ansprüche 8 bis 13, wobei mindestens einer von dem ersten und dem zweiten repräsentativen Leitfähigkeitwert ein gleitender Durchschnitt der empfangenen Leitfähigkeitswerte ist.

15. Verfahren nach einem der Ansprüche 8 bis 14, des Weiteren umfassend:
Empfangen von Temperaturwerten von einem Temperatursensor, der entlang des Fluidkreislaufs angeordnet ist; und
Anpassen des Leitfähigkeitswerts des mindestens einen Leitfähigkeitsdetektors basierend auf einer Temperatur, die von dem Temperatursensor gemessen wurde, und wobei der Leitfähigkeitsdetektor und der Temperatursensor gegebenenfalls ein einziger Sensor sind.

16. Verfahren nach einem der Ansprüche 8 bis 15, des Weiteren umfassend Bestimmen eines dritten Kalibrierungspunkts, wobei Berechnen der Kalibrierungskurve auf dem ersten, zweiten und dritten Kalibrierungspunkt basiert, und wobei Berechnen der Kalibrierungskurve des Weiteren eine nicht-lineare Polynomanpassung für den ersten, zweiten und dritten Kalibrierungspunkt umfasst.

17. Dialysesystem nach einem der Ansprüche 1 bis 7, oder Verfahren nach einem der Ansprüche 8 bis 16, wobei der mindestens eine Leitfähigkeitsdetektor mindestens eines von a), b) oder c) umfasst, wobei:
a) ein Leitfähigkeitsdetektor entlang des Dialysefluidkreislaufs an einer Stelle vorgeordnet zu einem Dialysator angeordnet ist;
b) ein Leitfähigkeitsdetektor entlang des Dialysefluidkreislaufs an einer Stelle nachgeordnet zu dem Dialysator angeordnet ist; und
c) ein Leitfähigkeitsdetektor entlang des Dialysefluidkreislaufs zwischen einem Salzinjektionsport in dem Dialysefluidkreislauf und einem Säurelösungsinjektionsport in dem Dialysefluidkreislauf angeordnet ist.

18. Dialysesystem nach einem der Ansprüche 1 bis 7 oder Verfahren nach einem der Ansprüche 8 bis 16, wobei das Fluid ein Dialysat ist.

## Revendications

1. Système de dialyse (2) comprenant :
au moins une pompe (93, 98, 100) disposée le long d'un circuit de fluide pour conduire un fluide à un débit de pompe à travers le circuit de fluide, le débit de pompe étant ajustable ;
un conductimètre comprenant une sonde (126) configurée pour être placée en communication fluidique avec le circuit de fluide pour mesurer les valeurs de conductivité du fluide ;
au moins un détecteur de conductivité (50, 52, 54) disposé le long du circuit de fluide pour mesurer des valeurs de capteur du fluide ;
un dispositif de commande configuré pour :
i) recevoir les valeurs de conductivité du conductimètre et les valeurs de capteur de l'au moins un détecteur de conductivité ;
ii) calculer une pluralité de valeurs de conductivité représentatives à partir des valeurs de conductivité reçues et une pluralité de valeurs de capteur représentatives à partir des valeurs de capteur reçues ;
iii) déterminer un premier point d'étalonnage (142, 144) associé à une première valeur de conductivité représentative et à une première valeur de capteur représentative ;
iv) déterminer un deuxième point d'étalonnage (142, 144) associé à une deuxième valeur de conductivité représentative et à une deuxième valeur de capteur représentative ; et
v) calculer une courbe d'étalonnage (152) sur la base des premier et deuxième points d'étalonnage.

2. Système de dialyse selon la revendication 1, le dispositif de commande étant configuré pour ajuster un débit de pompe de l'au moins une pompe jusqu'à ce que la première valeur de conductivité représentative soit dans un intervalle d'étalonnage avant que le dispositif de commande ne détermine le premier point d'étalonnage.

3. Système de dialyse selon la revendication 2, le dispositif de commande étant configuré pour introduire un délai après l'ajustement du débit de pompe et avant la détermination du premier point d'étalonnage, par exemple, le délai étant supérieur à 30 secondes.

4. Système de dialyse selon l'une quelconque des revendications ci-dessus, le dispositif de commande étant configuré pour ajuster un débit de pompe de l'au moins une pompe jusqu'à ce que la deuxième valeur de conductivité représentative soit dans un intervalle d'étalonnage (138, 140) avant que le dispositif de commande ne détermine le deuxième point d'étalonnage, et éventuellement le dispositif de commande étant configuré pour introduire un délai après l'ajustement du débit de pompe et avant la détermination du deuxième point d'étalonnage, par exemple, le délai étant supérieur à 30 secondes.

5. Système de dialyse selon l'une quelconque des revendications ci-dessus, au moins l'une des première et deuxième valeurs de capteur représentatives étant une moyenne mobile des valeurs de capteur reçues.

6. Système de dialyse selon l'une quelconque des revendications ci-dessus, comprenant en outre un capteur de température disposé le long du circuit de fluide, le dispositif de commande étant configuré pour ajuster la valeur de conductivité de l'au moins un détecteur de conductivité sur la base d'une température mesurée par le capteur de température.

7. Système de dialyse selon l'une quelconque des revendications ci-dessus, le dispositif de commande étant configuré pour déterminer un troisième point d'étalonnage, le dispositif de commande étant configuré pour calculer la courbe d'étalonnage sur la base des premier, deuxième et troisième points d'étalonnage, et éventuellement le dispositif de commande calculant la courbe d'étalonnage par l'intermédiaire d'un ajustement polynomial non linéaire des premier, deuxième et troisième points d'étalonnage.

8. Procédé d'étalonnage d'au moins un détecteur de conductivité disposé le long d'un circuit de liquide de dialyse, comprenant :
i) le passage d'un fluide à travers le circuit de fluide de dialyse en faisant fonctionner au moins une pompe (93, 98, 100) disposée le long du circuit de fluide de dialyse ;
ii) la réception de valeurs de conductivité provenant d'un conductimètre (126) disposé le long du circuit de fluide de dialyse, et de valeurs de capteur provenant de l'au moins un détecteur de conductivité (50, 52, 54) disposé le long du circuit de fluide de dialyse ;
iii) le calcul d'une pluralité de valeurs de conductivité représentatives à partir des valeurs de conductivité reçues, et d'une pluralité de valeurs de capteur représentatives à partir des valeurs de capteur reçues ;
iv) la détermination d'un premier point d'étalonnage (142, 144) associé à une première valeur de conductivité représentative et à une première valeur de capteur représentative ;
v) la détermination d'un deuxième point d'étalonnage (142, 144) associé à une deuxième valeur de conductivité représentative et à une deuxième valeur de capteur représentative ; et
vi) le calcul d'une courbe d'étalonnage (152) sur la base des premier et deuxième points d'étalonnage.

9. Procédé selon la revendication 8, comprenant en outre, avant de déterminer le premier point d'étalonnage, l'ajustement d'un débit de pompe de l'au moins une pompe jusqu'à ce que la première valeur de conductivité représentative soit dans un intervalle d'étalonnage (138, 140) .

10. Procédé selon la revendication 9, comprenant en outre l'introduction d'un délai après l'ajustement du débit de pompe et avant la détermination du premier point d'étalonnage, par exemple, le délai étant supérieur à 30 secondes.

11. Système de dialyse selon l'une quelconque des revendications 1 à 3 ou procédé selon l'une quelconque des revendications 8 à 10, le fluide étant une solution de concentration inconnue, par exemple, la solution comprenant un sel et éventuellement comprenant en outre un acide.

12. Procédé selon l'une quelconque des revendications 8 à 11, comprenant en outre, avant de déterminer le deuxième point d'étalonnage, l'ajustement d'un débit de pompe de l'au moins une pompe jusqu'à ce que la deuxième valeur de conductivité représentative soit dans un intervalle d'étalonnage, et éventuellement, le procédé comprenant en outre l'introduction d'un délai après l'ajustement du débit de pompe et avant la détermination du deuxième point d'étalonnage, par exemple, le délai étant supérieur à 30 secondes.

13. Système de dialyse selon l'une quelconque des revendications 2, 3 ou 4 ou procédé selon l'une quelconque des revendications 9 ou 12, l'intervalle d'étalonnage étant défini par un seuil supérieur et un seuil inférieur.

14. Procédé selon l'une quelconque des revendications 8 à 13, au moins l'une des première et deuxième valeurs de conductivité représentatives étant une moyenne mobile des valeurs de conductivité reçues.

15. Procédé selon l'une quelconque des revendications 8 à 14, comprenant en outre :
la réception de valeurs de température provenant d'un capteur de température disposé le long du circuit de fluide ; et
l'ajustement de la valeur de conductivité de l'au moins un détecteur de conductivité sur la base d'une température mesurée par le capteur de température, et éventuellement le détecteur de conductivité et le capteur de température étant un seul capteur.

16. Procédé selon l'une quelconque des revendications 8 à 15, comprenant en outre la détermination d'un troisième point d'étalonnage, le calcul de la courbe d'étalonnage étant sur la base des premier, deuxième et troisième points d'étalonnage, et éventuellement, le calcul de la courbe d'étalonnage comprenant en outre un ajustement polynomial non linéaire des premier, deuxième et troisième points d'étalonnage.

17. Système de dialyse selon l'une quelconque des revendications 1 à 7 ou procédé selon l'une quelconque des revendications 8 à 16, l'au moins un détecteur de conductivité comprenant au moins l'un des éléments parmi : a) b), ou c), avec :
a) un détecteur de conductivité disposé le long du circuit de liquide de dialyse à un emplacement en amont d'un dialyseur ;
b) un détecteur de conductivité disposé le long du circuit de liquide de dialyse à un endroit situé en aval du dialyseur ; et
c) un détecteur de conductivité disposé le long du circuit de liquide de dialyse entre un orifice d'injection de sel dans le circuit de liquide de dialyse et un orifice d'injection de solution acide dans le circuit de liquide de dialyse.

18. Système de dialyse selon l'une quelconque des revendications 1 à 7 ou procédé selon l'une quelconque des revendications 8 à 16, le fluide étant un dialysat.
